# EUROPEAN PATENT APPLICATION

(11) **EP 4 397 751 A1**
(43) Date of publication of application: **10.07.2024**
(21) Application number: 23305007.9
(22) Date of filing: 04.01.2023
(51) Int. Cl.: C12N 5/071

(54) **METHODS FOR THE PRODUCTION OF HUMAN MELANOCYTES FROM PLURIPOTENT STEM CELLS**

(71) Applicant: Centre d'Etude des Cellules Souches (CECS), 91100 Corbeil Essonnes (FR)
(72) Inventor: The designation of the inventor has not yet been filed
(74) Representative: Plasseraud IP

(57) **Abstract**

The present invention pertains to in vitro methods for obtaining a population of mature and functional human melanocytes from human pluripotent stem cells (hPSC) and to uses of said population. The method according to the present invention comprises a differentiation step wherein neural crest cells are cultured in presence of a GSK3 inhibitor such as CHIR99021 or WNT3A, BMP4, AA, SCF and EDN3.

## Description

### Field of the Invention

The present invention pertains to *in vitro* methods for obtaining a population of mature human melanocytes from human pluripotent stem cells (hPSC) and to uses of said population.

### Background of the Invention

Skin pigmentation disorders represent one of the most frequent cutaneous alterations. Vitiligo is the most common acquired depigmentation disorder, affecting more than 70 million people worldwide. Vitiligo is clinically characterized by depigmented macules resulting from the loss of functional melanocytes. Whereas this disease is often dismissed as a cosmetic problem, vitiligo can be psychologically devastating, and is associated with a considerable burden on daily life. Current therapies are limited, and no known treatment can consistently induce repigmentation in all patients. In addition, some patients are not responsive to medical treatments (Khaitan, B., Kathuria, S., & Ramam, M. (2012) Indian Journal of Dermatology Venereology and Leprology, 78(6), 715) and can only be treated with surgery. Surgical treatment of vitiligo consists in introducing melanocytes from the patient's pigmented skin regions into the depigmented lesions via different procedures (e.g., tissue grafts or cellular grafts). However, these techniques require surgical biopsy and can induce melanocyte depletion in the donor site.

Over the last decade, various protocols were developed for differentiating human pluripotent stem cells into melanocytes and provided significant improvements in the understanding of signaling pathways essential for melanocyte generation (Fang, D et al. (2006). Stem cells, 24(7), 1668-1677.; Ohta, Shigeki, et al. " Skin Stem Cells. Humana Press, Totowa, NJ, 2013. 193-215.; Nissan, Xavier, etal. Proceedings of the National Academy of Sciences 108.36 (2011): 14861-14866.; Mica, Yvonne, et al. Cell reports 3.4 (2013): 1140-1152.; Jones, Jennifer C., et al. The Journal of investigative dermatology 133.8 (2013): 2104.; Kawakami, Tamihiro, et al. Journal of Investigative Dermatology 138.1 (2018): 150-158; Catherine Cohen et al. Pigment Cell Melanoma Res. 2022 Dec 7.10.1111/pcmr.13077), hPSC-based studies further helped deciphering molecular mechanisms underlying several diseases associated with pigment defects (Lo Cicero, Alessandra, et al Scientific reports 8.1 (2018): 1-10.; Allouche, Jennifer, et al, Proceedings of the National Academy of Sciences 112.29 (2015): 9034-9039.; Mica, Yvonne, et al., Cell reports 3.4 (2013): 1140-1152.).

However, there is no report in the prior art of a method that would allow for differentiating a pure and homogenous population of functional melanocytes without any selection resulting in a large-scale melanocyte production. The efficient and reliable production of melanocytes with high proliferation capacity and stability over time in culture is nevertheless essential for developing drug screening protocols and cell therapies based on the use of hPSC-derived melanocytes.

There is thus an urgent need for novel methods for generating populations of mature human melanocytes from human pluripotent stem cells.

### Summary of the invention

The invention is defined by the claims.

The present invention pertains to an *in vitro* method for obtaining a population of human melanocytes, wherein said method comprises a differentiation step wherein neural crest cells are cultured in presence of a Glycogen Synthase Kinase 3 (GSK3) inhibitor such as CHIR99021 or Wingless-type family member 3A (WNT3a), Bone Morphogenic Protein 4 (BMP4), Ascorbic Acid (AA), Stem Cell Factor (SCF) and Endothelin 3 (EDN3).

The method according to the present invention particularly comprises the following steps:
a) an induction step wherein human pluripotent stem cells are cultured in a neural medium to obtain neural crest cells;
b) a differentiation step wherein the neural crest cells obtained in step a) are cultured in presence of a GSK3 inhibitor such as CHIR99021 or WNT3a, BMP4, AA, SCF and EDN3 to obtain melanocyte precursors;
c) a maturation step wherein the melanocyte precursors obtained in step b) are cultured in presence of at least one agent stimulating the maturation of melanocytes to obtain mature and functional human melanocytes; and
d) an amplification step wherein the melanocytes obtained in step c) are cultured on a medium suitable for melanocyte culture to obtain a population of human melanocytes.

This method can advantageously be carried out on a 2D or a 3D culture medium.

The present invention also pertains to the population of human melanocytes obtained from the above method and to uses thereof. The population of human melanocytes can advantageously be used in the treatment of diseases, including skin pigmentation disorders such as vitiligo. They can also be used as a cellular model, for screening candidate cosmetic and therapeutic compounds.

### Brief description of the drawings

**Figure 1****:** Characterization of melanocyte derived hPSCs: Diagram of the melanocytic differentiation from hPSCs (**A:** 3D protocol, **B:** 2D Protocol).
**Figure 2****:** Obtention of a pure population of melanocytes at passage 0 and their maturation by flow cytometry. TYRP1 expression by FACS analysis of Mel-hPSC at different passages (A: 3D protocol, B: 2D Protocol).
**Figure 3****:** Long-term proliferation capacity of mel-hPSC (3D protocol): **A.** Doubling time measurements during the growing phase of the Mel-hPSC, from Passage 3 (P3) to Passage 26 (P26); **B.** Cumulated cell expansion factor calculated from Passage 3 (P3) to Passage 15 (P15).

### Detailed description of the invention

The present inventors have developed a step-by-step protocol including a phase of induction, differentiation, maturation, and amplification which allow for the standardized production of mature human melanocytes. The method according to the present invention is a multi-step protocol which allows reproducing the main stages of melanocyte development, as confirmed by transcriptomic analyses. The inventors have shown that the melanocytes obtained are fully mature and functional: they contain melanosomes, produce melanin, and are able to integrate the epidermal basal layer when introduced into immunodeficient mice. The melanocytes generated are further capable of long-term proliferation in culture without losing their original phenotype. The results shown herein represent a meaningful, broadly applicable and long-term advance for the treatment of pigmentation disorders and opens up new perspectives for the development of innovative biotherapies for skin pigmentation diseases.

The method according to the present invention is an *in vitro* method for obtaining a population of human melanocytes, wherein said method comprises a differentiation step wherein neural crest cells are cultured in presence of GSK3 inhibitor such as CHIR99021 or WNT3A, BMP4, AA, SCF and EDN3.

The method according to the present invention particularly comprises the following steps:
a) an induction step wherein human pluripotent stem cells are cultured in a neural medium to obtain neural crest cells;
b) a differentiation step wherein the neural crest cells obtained in step a) are cultured in presence of GSK3 inhibitor such as CHIR99021 or WNT3A, BMP4, AA, SCF and EDN3 to obtain melanocyte precursors;
c) a maturation step wherein the melanocyte precursors obtained in step b) are cultured in presence of at least one agent stimulating the maturation of melanocytes to obtain mature human melanocytes;
d) an amplification step wherein the melanocytes obtained in step c) are cultured on a medium suitable for melanocyte culture to obtain a population of human melanocytes.

Advantageously, the method according to the present invention can be either a 2D method or a 3D method, meaning that it can be performed either in a 2D cell culture environment or a 3D cell culture environment. **"2D cell culture environment"** refers to methods wherein the cells are cultured on a flat surface such as the bottom of a petri dish, plate, well or flask, and in which cells grow as a monolayer. In the context of the present invention the method can advantageously be performed in 2D by using petri dishes. On the other hand, a **"3D cell culture environment"** refers to culture conditions in which cells are permitted to grow or interact with their surroundings in all three dimensions. 3D models can be divided into: i) suspension cultures on non-adherent plates, ii) cultures in concentrated medium or in gel-like substances and iii) cultures on a scaffold (see Kapa 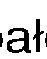 czyńska et al. *Archives of Medical Science* 14.4 (2018): 910-919). 3D culture methods can e.g. be performed by culturing the cells in a culture medium placed in a cell culture bag, in the wells of a well plate or in a bioreactor. Culture bags and bioreactors useful this context are plentiful and can be easily obtained. According to a preferred embodiment, the 3D cell culture environment is the well of a 96-well plate.

The term **"melanocytes"** has its general meaning in the art. Melanocytes are pigment-producing cells responsible for coloration of skin, eyes, and hair. In vertebrate development, melanocytes originate from the neural crest cells and undergo a process of fate-specification, proliferation, migration, survival, and differentiation before finally residing in the epidermis. Pigmentation is achieved via a process called melanogenesis during which the melanin pigment is produced in specialized organelles called melanosomes which are then transported along the dendrites and transferred to growing hair, or surrounding keratinocytes to play a critical role in protecting human tissues from the deleterious effects of sun's light ultraviolet (UV) radiations. Melanocytes molecularly are recognizable by identification of melanocyte-specific proteins as tyrosinase (TYR), tyrosinase-related protein 1 and 2 (TYRP1, TYRP2/DCT), melanosomal matrix proteins (Pmel17/GP100), microphthalmia transcription factor (MITF) (Cichorek, Miros 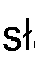 awa, et al. *Advances in Dermatology and Allergology*/*Post* 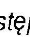 *py Dermatologii i Alergologii* 30.1 (2013): 30-41). The appearance of tyrosinase, the enzyme of melanin synthesis, is the main characteristic of final melanocyte maturation. **"Melanocyte precursors"** express melanogenesis markers such as MITF or DCT, but do not express tyrosinase.

A **"population"** of human melanocytes refers to a homogenous group of cells wherein the majority (e.g. at least about 80%, preferably at least about 90%, more preferably at least about 95%, even more preferably at least 99%) of the total number of cells have the characteristics of human primary melanocytes.

Step a) of the method according to the present invention consists in culturing human pluripotent stem cells on a neural medium so as to obtain neural crest cells.

As used herein, the term **"human pluripotent stem cell"** or **"hPSC"** refers to any human precursor cell that has the ability to form any adult cell. In a particular embodiment, human pluripotent stem cells include but are not limited to human embryonic stem cells (hES cells) or human induced pluripotent stem cells (hiPS cells).

In a preferred embodiment, said human pluripotent stem cells are obtained without destruction of human embryos.

As used herein, the term **"human embryonic stem cells"** or **"hES cells"** or **"hESCs"** refers to human precursor cells that have the ability to form any adult cell (see for review Löser, Peter, et al. Stem cells 28.2 (2010): 240-246). Human embryonic stem cells useful in the context of the present invention can be obtained from any cell line, such as the SA-01 (Cellartis, Götenborg Sweden) cell line.

As used herein, the term **"human induced pluripotent stem cells"** or **"human iPS cells"** or **"human iPSCs"** or **"hiPSCs"** refers to a type of human pluripotent stem cell artificially derived from a human non-pluripotent cell (e.g. an adult somatic cell). Human induced pluripotent stem cells are identical to human embryonic stem cells in the ability to form any adult cell, but are not derived from an embryo. For review on protocols allowing for the production of iPSCs, one can consult Shi, Yanhong, et al. Nature reviews Drug discovery 16.2 (2017): 115-130. According to an embodiment of the invention human iPS cells may be selected from any human iPS cell line. An Example of human iPS cell line useful according to the present invention includes cell line hiPSC-1503 which has been reprogrammed from dermal fibroblasts and grown as described in Guenou et al (The Lancet 374.9703 (2009): 1745-1753).

In the context of the present invention, hPSCs are submitted to an induction step so as to obtain neural crest cells.

The **"induction step"** according to the present invention refers to the derivation of neural crest cells from hPSCs.

The term **"neural crest cells"** or **"NCCs"** has its general meaning in the art. It refers to multipotent and migratory cells that arise from the embryonic ectoderm germ layer and contribute to the formation of a wide range of cell lineages including melanocytes craniofacial cartilage and bone (see Le Douarin and Dupin, Curr. Opin. Genet. Dev. (2003) 13, 529-536)*.* NCSCs markers include genes involved in specific aspects of NCCs fate. These markers include Sox10, p75NTR, Ret, and Pax7 (Yang et al. Disease Markers 2020 (2020). During embryonic development, NCCs are specified at the neural plate border (NPB) region before they undergo an epithelial-mesenchymal transition and migrate out of the neural tube. NCC induction at the NPB relies on BMP, WNT, Notch/Delta, and FGF signaling emanating from the surrounding embryonic tissues (see Rogers et al., Wiley Interdiscip. Rev. Dev. Biol. (2012) 1, 52-68). Several protocols have been developed to derive NCCs from hPSCs (see Srinivasan and Yi-Chin Toh. Frontiers in molecular neuroscience 12 (2019): 39).

In the context of the present invention, said induction step is carried out by means of a neural medium. The **"neural medium"** refers to any culture medium that allows for the induction of neural crest cells from hPSCs. As mentioned above, several protocols have been developed to derive NCCs from hPSCs. Efficient NCC induction can be e.g. initiated by culturing hPSCs on a culture medium supplemented with:
- an agent activating the canonical Wnt pathway such as a GSK3 inhibitor selected from CHIR-99021 and WNT3a when a 2D culture environment is used; or
- an agent activating the canonical Wnt pathway such as a GSK3 inhibitor selected from CHIR-99021 and WNT3a, and small molecules inhibiting the BMP and the TGFβ pathways, such as LDN193189 and SB431542, respectively, when a 3D culture environment is used.

A suitable culture medium that can be used as the neural medium further supplemented with the above agents can e.g. comprise the neurobasal and Gibco^{™} Ham's F12 Media (Thermo Fisher Scientific) complemented with 2% of B-27 without Vitamin A (Invitrogen) and with N-2 (Invitrogen).

An **"agent activating the canonical WNT pathway"** has its general meaning in the art. Wnt signaling is a critical component during embryonic development and also plays an important role in regulating adult tissue homeostasis. The most prominent Wnt signaling activators focus on inhibiting the glycogen synthase kinase 3 (GSK3), which normally disrupts the β-catenin destruction complex, allowing transportation of β-catenin into the nucleus to participate in gene transcription and expression. GSK3 inhibitors are thus preferred agents to be used for activating the canonical WNT pathway. **"GSK3 inhibitors"** are a well-defined class of compounds that has been extensively studied in the prior art. For review, one can consult Cohen and Goedert (Nature reviews Drug discovery 3.6 (2004): 479-487), Tran and Jie (Protein Science 26.4 (2017): 650-661) or Chen et al. (Proceedings of the National Academy of Sciences 111.14 (2014): 5349-5354) which list several compounds falling under this definition. Preferred GSK3 inhibitors according to the present invention are WNT3a or Chiron GSK3 inhibitors such as CHIR99021.

**"CHIR99021"** or "**6-((2-((4-(2,4-Dichlorophenyl)-5-(4-methyl-1*H*-imidazol-2-yl) pyrimidin-2-yl)amino)ethyl)amino)nicotinonitrile"** (CAS number: 252917-06-9) is a chemical compound which acts as an inhibitor of the enzyme GSK-3. It can be easily obtained from various specialized manufacturers.

**"Wnt Family Member 3A"** or **"WNT3a"** is a protein that in humans is encoded by the WNT3A gene. Its amino acid sequence is accessible under reference P56704 in the UniProt database. It can be easily obtained from various specialized manufacturers.

**"LDN193189"** or **"4-{6-[4-(Piperazin-1-yl) phenyl]pyrazolo[1,5-a]pyrimidin-3-yl}quinoline"** (CAS number: 1062368-24-4) is a chemical compound that is a potent inhibitor of the bone morphogenetic (BMP) pathway. It can be easily obtained from various specialized manufacturers.

**"SB431542"** or "**4-[4-(2*H*-1,3-Benzodioxol-5-yl)-5-(pyridin-2-yl)-1*H*-imidazol-2-yl]benzamide"** (CAS number: 301836-41-9) is a chemical compound that inhibits the TGF-β/Activin/NODAL pathway. It can be easily obtained from various specialized manufacturers.

In the context of the present invention, hPSCs are cultured over a period of time sufficient for obtaining NCCs. Typically, hPSCs are cultured in the neural medium during 3 to 7 days, preferably 4 to 6 days. Accordingly, step a) of the method according to the present invention is preferably carried over 3 to 7 days. More particularly, the induction step is preferably carried over 6 to 7 days when the culture environment used is a 2D culture environment, and over 3 to 4 days when the culture environment used is a 3D culture environment.

hPSCs are typically seeded at a concentration of 20 000 to 40 000 cells per cm² when the culture environment used is a 2D culture environment, and at a concentration of 8 000 to 12 000 cells per well when the culture environment used is a 3D culture environment which is a 96-well plate.

Advantageously, when the culture environment is a 2D culture environment, the surface of the support used, i.e. the surface of the plate, dish, etc... can be coated with a basement membrane matrix to allow for the cell to properly adhere and grow on the culture surface. An appropriate basement membrane matrix useful in the context of the present invention is e.g. the Matrigel^{®} matrix produced by Corning Life Sciences.

Typically, during the induction step, the agent activating the canonical Wnt pathway, in particular CHIR-99021 or WNT3a, preferably CHIR-99021, is present in a concentration of about 2 to 4 µM, preferably of about 3 µM.

Typically, during the induction step, LDN193189 is present in a concentration of about 0,1 to 0,3 µM, preferably of about 0,2µM.

Typically, during the induction step, SB431542 is present in a concentration of about 30 to 50 µM, preferably of about 40µM.

When the culture environment used is a 2D culture environment, the method according to the present invention can advantageously comprise a step a'), wherein the neural crest cells obtained in step a), i.e. at the end of the induction step, are enzymatically sorted with Accutase^{®} or trypsin prior to step b). Such an additional step allows for the dissociation, for the detachment of the cells prior to the differentiation step.

Accutase^{®} is an enzyme mixture with proteolytic and collagenolytic enzyme activity: it mimics the action of trypsin and collagenase at the same time. It is easily obtained from specialized manufacturers.

Trypsin (CAS number: 9002-07-7) is a serine protease secreted in the first section of the small intestine so as to start the digestion of protein molecules. Trypsin allows resuspending cells adherent to the cell culture dish wall during *in vitro* cell culturing. It is easily obtainable from various manufacturers.

When the culture environment used is a 3D culture environment, the hPSCs can form embryoid bodies (EB), i.e. three-dimensional aggregates of the pluripotent stem cells, which contain the neural crest cells. Said EBs can then be plated on a suitable culture medium to allow for the neural crest cells to exit them.

The method according to the present invention is characterized by the fact that is comprises a differentiation step wherein neural crest cells are cultured in presence of a GSK3 inhibitor such as CHIR99021 or WNT3a, BMP4, AA, SCF and EDN3.

The **"differentiation step"** according to the present invention refers to the formation of melanocyte precursors from neural crest cells. As mentioned above, melanocyte precursors are melanocytes which express melanogenesis markers such as MITF, DCT or TYRP1 but do not express tyrosinase. Differentiation of neural crest cells into melanocyte precursors is performed by culturing the NCCs on a culture medium suitable for melanocyte culture, i.e. survival and proliferation, referred herein as a "melanocyte medium", further comprising the specific agents CHIR99021 or WNT3a, BMP4, AA, SCF and EDN3.

The **"melanocyte medium"** or **"medium suitable for melanocyte culture"** is any physiologically acceptable medium that allows for the growth of melanocytes. Physiologically acceptable media are aqueous media that comprise electrolytes such as sodium potassium, magnesium and/or calcium salts, including anions such as chloride, carbonate, hydroxide or caprylate. Suitable culture medium according to the present invention typically comprise Bovine pituitary exctact, fetal bovine serum, recombinant human insulin-like growth factor-1, bovine transferrin, basic fibroblast growth factor, hydrocortisone, heparin, phorbol 12-myristate 13-acetate, rh-insulin. Suitable culture media that can be used as a melanocyte medium for performing the method according to the present invention are e.g. the Lonza^{™} MGM^{™}-4 Melanocyte Growth Medium-4 commercialized by Thermo Fisher Scientific or the 254CF Human Melanocyte Basal Medium (Invitrogen).

According to the present invention, the **"differentiation medium"** comprises a melanocyte medium supplemented with CHIR99021 or WNT3a, BMP4, AA, SCF and EDN3.

The term **"BMP4"** refers to **"Bone Morphogenetic Protein 4".** BMP4 is a polypeptide belonging to the TGF-β superfamily of proteins. Its amino acid sequence is accessible under reference P12644 in the UniProt database. Human recombinant BMP4 can be easily obtained from various specialized manufacturers.

**"Ascorbic acid"** or "**(5*R*)-[(1*S*)-1,2-Dihydroxyethyl]-3,4-dihydroxyfuran-2(5*H*)-one**" (CAS number: 50-81-7) is an organic compound which is essential for humans and many animals. It is used in countless applications. Ascorbic acid is very easily accessible from multiple specialized manufacturers.

**"Stem Cell Factor"** or **"SCF"** (also known as KIT-ligand or steel factor) is a cytokine that binds to the c-KIT receptor. Its amino acid sequence is accessible under reference P21583 in the UniProt database. It can be easily obtained from various specialized manufacturers.

**"EDN3"** or **"Endothelin-3"** is a protein that in humans is encoded by the EDN3 gene. Its amino acid sequence is accessible under reference P14138 in the UniProt database. It can be easily obtained from various specialized manufacturers.

In the context of the present invention, neural crest cells are cultured over a period of time sufficient for obtaining melanocyte precursors.

Typically, when the culture environment used is a 2D culture environment, neural crest cells are cultured during 10 to 15 days to obtain a population of melanocyte precursors. According to this embodiment, step b) is therefore carried over 10 to 15 days, preferably over about 14 days.

According to another embodiment, when the culture environment used is a 3D culture environment, neural crest cells are typically cultured during 20 to 28 days to obtain a population of melanocyte precursors. According to this embodiment, step b) is therefore carried over 20 to 28 days, preferably over about 25 days.

Typically, during the differentiation step, CHIR-99021, is present in a concentration of about 2 to 4 µM, preferably of about 3 µM.

According to a specific embodiment, when the culture environment used is a 3D culture environment, CHIR-99021 can advantageously be present in a concentration of about 2 to 4 µM, preferably of about 3 µM during the first 3 to 4 days of differentiation, and then its concentration is lowered to about 0,4 to 0,6 µM, preferably about 0,5 µM, for the rest of the differentiation step.

Typically, during the differentiation step, BMP4 is present in a concentration of about 10 to 12 pM, preferably of about 10,9 pM.

Typically, during the differentiation step, AA is present in a concentration of about 0,2 to 0,4 mM, preferably of about 0,3 mM.

Typically, during the differentiation step, SCF is present in a concentration of about 40 to 60 ng/mL, preferably of about 50 ng/mL.

Typically, during the differentiation step, EDN3 is present in a concentration of about 80 to 120 nM, preferably of about 100nM.

Advantageously, the culture medium can be replaced with a new culture medium after a few days of culture. The culture medium used as a replacement (also referred to as "new" culture medium) is the same as that described above for the differentiation step, i.e. the melanocyte medium supplemented with CHIR99021 or WNT3a, BMP4, AA, SCF and EDN3. Such a replacement is generally performed once or several times, preferably every 2 to 3 days during the differentiation step,

According to a specific embodiment, the method according to the present invention can advantageously comprise a step b'), wherein the melanocyte precursors obtained in step b) are enzymatically sorted with Accutase^{®} or trypsin prior to step c). Enzymatic sorting allows for the detachment/dissociation of the cells prior to the maturation step.

The method according to the present invention further comprises a maturation step wherein the melanocyte precursors obtained in the differentiation step are cultured in presence of at least one agent stimulating the maturation of melanocytes to obtain mature human melanocytes.

The **"maturation step"** according to the present invention refers to the formation of mature and functional melanocytes (i.e. melanocytes that are directly derived from melanocyte precursors) from melanocyte precursors. It thus refers to the obtention of mature, i.e., fully competent, melanocytes that express all the melanogenesis markers including tyrosinase. Maturation of melanocyte precursors into functional melanocytes is performed by culturing the melanocyte precursors on a melanocyte medium as defined above in presence of at least one agent stimulating the maturation of melanocytes.

In the context of the present invention, maturation is therefore performed by means of a **"maturation medium",** which corresponds to a melanocyte medium supplemented with at least one agent stimulating the maturation of melanocytes.

According to an alternative embodiment, the maturation medium can also comprise one or more of the molecules used for stimulating melanocyte differentiation during the differentiation step, i.e. CHIR99021 or WNT3a, BMP4, AA, SCF and EDN3.

According to a specific embodiment, the maturation step can be performed by culturing the melanocyte precursors on the same medium as that used for the differentiation step, i.e. the differentiation medium, but further supplemented with said at least one agent stimulating the maturation of melanocytes. According to this embodiment, the "maturation medium" used therefore comprises a melanocyte medium, further supplemented with CHIR99021 or WNT3a, BMP4, AA, SCF, EDN3 and the agent stimulating the survival and maturation of melanocytes.

The concentrations of CHIR99021 or WNT3a, BMP4, AA, SCF and EDN3 used according to these two embodiments are similar to those used in the context of the differentiation step.

The skilled person knows several **"agents stimulating the survival and maturation of melanocytes"** that can be used in the context of the present invention. Agents stimulating the maturation of melanocytes are typically selected from activators of the cAMP pathway such as forskolin, cholera toxin, cyclic AMP factor, Phorbol 12-myristate 13 acetate (PMA), hydrocortisone or the alpha-melanocyte-stimulating hormone (α-MSH). According to a preferred embodiment, the agent stimulating the survival and maturation of melanocytes used in the context of the present invention is an activator of the cAMP pathway, preferably forskolin.

**"Forskolin"** (CAS number: 66575-29-9) is a diterpene commonly used in *in vitro* cell cultures to increase cAMP levels by activating the enzyme adenylyl cyclase. It can be easily obtained from specialized manufacturers.

Typically, during the maturation step, the agent stimulating the maturation of melanocytes, in particular forskolin, is present in a concentration of about 15 to 25µM, preferably of about 20pM.

In the context of the present invention, the melanocyte precursors are cultured over a period of time sufficient for obtaining mature melanocytes. Typically, the maturation step is carried over 5 to 20 days, preferably over 7 to 14 days.

The culture medium used for the maturation step can also be advantageously replaced with a new culture medium after a few days of culture. The culture medium used as a replacement is the same as that described above for the maturation step, i.e. a maturation medium. Such a replacement is generally performed once or several times, preferably every 2 to 3 days during the maturation step.

In the context of the present invention, the cells can particularly be passaged, particularly during the maturation and amplification steps. This procedure consists not only in replacing the culture medium, but also in harvesting the cells from the culture medium and seeding these cells on a new culture medium so as to start a new culture. The new culture medium is identically defined as that on which the cells were cultured when harvested. This procedure may be necessary when the cells proliferate and reach 80%, 90% or even 100% confluence. In the context of the present invention, the cells can e.g. be passaged once or twice during the maturation step, and from 1 to 30 times, preferably at least 20 times during the amplification step.

Furthermore, when the cell culture environment used is a 2D culture environment, the cells can advantageously be enzymatically treated with a gentle cell dissociation reagent such as diluted trypsin or Accutase^{®} to allow for their detachment prior to the amplification step.

The method according to the present invention finally comprises an **"amplification step"** which refers to the growth of the mature melanocytes obtained in the maturation step on a melanocyte medium so as to allow for their proliferation and for the obtention of a population of human mature and functional melanocytes.

The melanocyte medium used in the context of this step is as defined above. According to the present invention, the melanocyte medium used in the amplification step is not supplemented with the agents listed above, i.e. does not comprise CHIR99021, WNT3a, BMP4, AA, SCF, EDN3 or forskolin.

The amplification step is carried over a period sufficient for reaching a desired size of population of human melanocyte. Typically, the amplification step comprises passaging the cells 20 to 30 times, each passaging corresponding to 7 to 10 days of culture.

According to a specific embodiment, the method according to the present invention is performed in a 2D culture environment and comprises the following steps:
a) culturing human pluripotent stem cells at a concentration of 20 000 to 40 000 cells per cm² on a neural culture medium comprising CHIR99021 during 3 to 7 days to obtain neural crest cells;
a') enzymatically sorting the neural crest cells obtained in step a);
b) culturing the neural crest cells sorted in step a') on the neural culture medium further comprising BMP4, ascorbic acid, Stem Cell Factor (SCF) and EDN3 during 10 to 15 days to obtain melanocyte precursors;
b') enzymatically sorting the melanocyte precursors obtained in step b);
c) culturing the melanocyte precursors sorted in step b') on the culture medium used in step b) further comprising forskolin and passaging the cells once or twice to obtain mature human melanocytes; and
d) culturing the melanocytes obtained in step c) on a medium suitable for melanocyte culture during at least 20 passages to allow for their proliferation/amplification and obtain a population of human melanocytes.

According to another specific embodiment, the method according to the present invention is performed in a 3D culture environment and comprises the following steps:
a) culturing human pluripotent stem cells on a 96-well plate at a concentration of 8 000 to 12 cells per well in a neural culture medium comprising CHIR99021, LDN193189 and SB431542 during 3 to 7 days to form embryoid bodies (EB) containing neural crest cells;
b) plating the embryoid bodies obtained in step in step a) to allow the neural crest cells to exit said embryoid bodies and then culturing during 20 to 25 days said neural crest cells in a medium suitable for melanocyte culture further comprising CHIR99021, BMP4, ascorbic acid, Stem Cell Factor (SCF) and EDN3 to obtain melanocyte precursors;
b') enzymatically sorting the melanocyte precursors obtained in step b);
c) culturing the melanocyte precursors sorted in step b') on the culture medium used in step b) further comprising forskolin to obtain mature and functional human melanocytes; and
d) culturing the melanocytes obtained in step c) on a medium suitable for melanocyte culture during at least 20 passages to allow for their proliferation and obtain a population of human melanocytes.

The method according to the present invention is typically carried out at a temperature of around 37°C.

The method according to the present invention allows obtaining fully mature and functional melanocytes that can be used in various applications. Therefore, according to a specific embodiment, the present invention also pertains to a population of human melanocytes, wherein said population is obtainable by the method defined above. The population of melanocytes according to the present invention has the specific characteristic of being extremely pure: around 99% of the total number of cells comprised in said population have the characteristics of human primary melanocytes. Accordingly, the population of human melanocytes according to the present invention does not need to be screened prior use: it can be directly used for therapeutic applications. The population of melanocytes according to the invention further shows an extremely high and sustained proliferative ability: it is extremely stable over time.

The population of melanocytes according to the present invention can be advantageously used for the treatment of multiple pathologies, notably in the treatment of pathologies associated with melanocyte deficiencies. Thus, according to a specific embodiment, the present invention pertains to a population of human melanocytes obtainable by the method defined above for use in the treatment of pathologies associated with melanocyte deficiencies.

As used herein, the expression "pathologies associated with melanocyte deficiencies", or "pigmentation disorders", refers to any pathology or disease in which there is a lack of functional melanocytes. This can be due to a deficiency in the enzymes necessary for the synthesis of melanin, to a deficiency in the development and/or proliferation and/or survival of melanocytes.

Pathologies associated with a melanocyte deficiency, or pigmentary disorders involving melanocytes, include, but are not limited to, diseases affecting the development of melanocytes from the neural crest (piebaldism, Waardenburg syndrome and dyschromatosis symmetrica hereditaria), diseases with defects in melanin synthesis (albinism), disorders of melanosome maturation or transfer (Hermansky-Pudlak syndrome, Chediak-Higashi syndrome and Griscelli syndrome) and vitiligo. According to a preferred embodiment, the pathology associated with melanocyte deficiencies treated in the context of the present invention is a skin pigmentation disorder such as vitiligo. Vitiligo is a de-pigmented patch on the skin. It occurs where the immune system has destroyed a patch of melanocytes.

A related aspect of the invention relates to a method for treating a subject suffering from a pathology associated with melanin deficiencies, said method comprising a step of administering to the subject an efficient amount of the population of human melanocytes according to the present invention.

The population of human melanocytes according to the present invention can also be used for the treatment of skin lesions, i.e. disruptions in skin function leading to the entry of pathogens, such as e.g. after a burn injury. Indeed, significant skin pigmentation changes occur when patients suffer lesions such as deep burn injuries. These pigmentation disorders may cause not only cosmetic and psychological issues, but also increase the risk of photoaging and, more importantly, the risk of developing skin cancer. In these patients, due to the lack of normal melanocytes, large amounts of autologous skin grafts are required. The population of human melanocytes according to the present invention can therefore advantageously be used in the treatment of such skin lesions.

In the context of the invention, the term "treating" or "treatment", refers to a method that is aimed at delaying or preventing the onset of a pathology, at reversing, alleviating, inhibiting, slowing down or stopping the progression, aggravation or deterioration of the symptoms of the pathology, at bringing about ameliorations of the symptoms of the pathology, and/or at curing the pathology.

As used herein, the term "efficient amount" refers to any amount of the population of human melanocytes according to the present invention that is sufficient to achieve the intended purpose.

The population of human melanocytes of the invention may be administered to a subject using any suitable method.

The population of human melanocytes according to the present invention can also advantageously be used as a cellular model to study e.g. melanocyte functions or pharmacological or toxicological applications. The present invention therefore also pertains to the use of the population of human melanocytes according to the present invention as a cellular model. Such a cellular model can advantageously be used in high content screening (HCS) and high throughput screening (HTS) assays. For instance, these assays can serve to identify therapeutic targets for disorders associated with melanocytes. Other assays can be performed in order to identify cosmetically active substances or compounds which modulate skin pigmentation.

The present invention therefore encompasses a method for screening candidate cosmetic or therapeutic compounds, wherein said method comprises the following steps:
i) Culturing the population of human melanocytes according to the present invention in presence of a candidate compound;
ii) Assaying the effects of said candidate compound on said population of human melanocytes; and
iii) Selecting said compound based on said effects.

Typically, the "effect" as used herein, can refer either to a desired (for example, stimulation of melanin synthesis) or an undesirable effect (such as toxicity) of a compound which is being screened for a therapeutic and/or cosmetic purpose. The skilled person in the art will understand what type of biological effect is to be assayed, according to the specific goal of the screening method.

The invention will now be further illustrated by means of the following examples. These examples are provided for illustrative purposes only and cannot be interpreted as limiting the scope of the invention.

### Examples

### Example 1: 3D Method for the large-scale production of melanocytes derived from pluripotent stem cells

### MATERIALS AND METHODS

**Cell culture. hPSC such as** hESC from one cell line, SA-01 (Cellartis, Götenborg Sweden) are grown as previously described (Guenou et al., 2009). For differentiation, embryoid bodies (EBs) are formed from hPSC clumps and growth on low attachment dishes in neural medium composed of neurobasal and Ham's F12 (ratio 1:1) complemented with 2% of B-27 without Vitamin A (Invitrogen) and with 1% of N-2 (Invitrogen). Neural crest induction was performed by using CHIR-99021(3µM) (TOCRIS), LDN-193189 (0,2 µM) (Miltenyi) and SB 431542 (40pM) (TOCRIS). EBs were grown in the same medium for 4 days. At day 4, neural medium was supplemented with 3µM CHIR99021, 100nM EDN3 (American peptide), 50ng/mL SCF (peprotech), 0.02nM of human recombinant BMP-4 (peprotech) and 0.3mM ascorbic acid (Sigma-Aldrich). From day 7 to day 30, EBs were plated on gelatine 0.1% and grown in MGM-4 medium (MGM-4 BulletKit Lonza) supplemented with 0.5µM CHIR99021, 100nM EDN3, 50ng/mL SCF, 0.02nM of human recombinant BMP-4 and 0.3mM ascorbic acid. Medium was changed every 2-3 days. Cells were dissociated using trypsin 0,05% (Invitrogen) and grown in the same medium supplemented with 20µM Forskolin (sigma) during 2 passages. From Passage 2, only MGM-4 media was used to growth the pigmented melanocytes. Cells were passaged at least 20 passages. Primary human epidermal melanocytes (HEM) (promocell) were grown in MGM-4 medium

**Quantitative RT-PCR (Q-PCR).** Total RNA was isolated from hPSC and HEM using RNeasy plus Mini extraction kit on Qiacube (Qiagen) according to the manufacturer's protocol. A total of 500 ng of RNA was used for reverse transcription using the Superscript III reverse transcription kit (Invitrogen). Q-PCR analysis was performed using a Quantstudio and Luminaris Color HiGreen qPCR Master Mixes Low Rox (Invitrogen) following the manufacturer's instructions. Quantification of gene expression was based on the DeltaCt Method and normalized on 18S expression.

**Immunocytochemistry.** Cells were fixed in 4% paraformaldehyde before permeabilization and blocking in PBS supplemented with 0.1% Triton X-100 and 1% BSA (Sigma-Aldrich). Primary antibodies were incubated 1:200 mouse anti-MITF (DAKO; M3621), 1:100 mouse anti-TYRP1 (Lsbio; LSC39939), 1:100 mouse anti-PMEL17 (abcam; ab15228), 1:100. then secondary antibodies were incubated with DAPI (ThermoFisher; D1306): Goat anti-mouse 555 (Invitrogen A-21422). Images were captured using a Zeiss epifluorescence illumination microscope.

**Flow cytometry.** Cells were fixed and permeabilized in FCM permeabilization buffer (Santa-Cruz biotechnology) at RT for 4 minutes. Cells were then washed with PBS and incubated at RT for 1 hour in 0.1% FBS with 1/100. dilution of mouse anti-TYRP1 antibody conjugated AF488 (Novusbio; NBP2-34720). Isotype specific IGg2a-AF488 were used as control (Novusbio; IC003G). Data were acquired on the MACSQuant Analyzer 10 (Miltenyi Biotech) and analyzed using FlowJo software (FlowJo LLC).

**Western blotting.** Cell pellets were lysed in RIPA lysis buffer (Sigma-Aldrich) supplemented with anti-proteases (Sigma-Aldrich) and anti-phosphatase (Roche). Protein concentrations were quantified by pierce BCA protein assay (Thermo scientific). Western blotting was performed by standard techniques using 4-12% NuPAGE Novex or 3-8% Bis-Tris gels (Invitrogen) and nitrocellulose membranes (Whatman, GE Healthcare). Blots were blocked in 5% milk in PBS 0.1% Tween 100, incubated with 1:500 of anti-Pax3 (DSHB), 1:500 of rabbit anti-MITF (ab20663, Abcam) and 1:1000 of mouse anti-Tyrosinase (ab738, Abcam), followed by 1:10000 of secondary antibody. Quantitative normalization was achieved with incubation with 1:50000 β-actin (A-3854, Sigma). Immunoreactive bands were revealed using Amersham ECL Plus Western Blotting Detection Reagents (GE Healthcare).

**Organotypic cultures and grafting onto mice.** Organotypic epidermis was generated as previously described (Guenouet al., 2009). Briefly, bioengineered skin equivalents were generated with fibrin matrix populated with human neonatal fibroblasts. Primary keratinocytes and HEM/Mel-hPSC with a ratio of 1:1 was seeded on the fibrin matrix, grown immersed to confluence for 10 days in FAD medium supplemented with SCF (10ng/mL), EDN3 (100nM), FSK (20µM) and αMSH trifluoroacetate 0.1µM (sigma). Bioengineered skin equivalents were transferred into PET inserts (Falcon) and have been put at the air-liquid interface for 14 days in FAD medium supplemented with SCF (10ng/mL), EDN3 (100nM), FSK (20µM), αMSH trifluoroacetate 0.1µM (sigma) and 50 µg/mL ascorbic acid. The others have been grafted onto the back of 12-week-old male Swiss mice (Charles River Laboratories), as previously described (L. Martínez-Santamaría, Del Rio, 2012). Implants were harvested 2 weeks after grafting, and the tissue specimens were fixed in 10% buffered formalin for paraffin embedding. The grafting protocol has been approved and authorized by CIEMAT.

### Staining on paraffin embedded tissue section

**For histology,** paraffin sections were stained with hematoxylin and eosin (H&E) using standard protocols.

**Fontana Masson staining:** Paraffin embedded sections were stained using Melanin Staining Kit (abcam; ab150669) according to manufacturer's protocol. **Immunohistochemistry:** Experiments were performed using Ventana BenchMark XT according to the manufacturing datasheets. Sections were stained using antibodies mouse anti-Involucrine (Sigma; I9018) and 1:100 rabbit anti TYRP1 (Lsbio; LSB4011). Slides were then incubated at RT with the appropriate secondary antibodies.

**Melanin quantification.** The cell pellets were resuspended in 1N NaOH and placed for 1 hour at 65°C. Absorbance was measured at 405nm (Clariostar; BMGLabtech). Melanin contents were measured according to a linear regression line obtained with graduated concentration of synthetic melanin. **Electron Microscopy (EM).** Cells were seeded on culture plate and fixed with 2% (vol/vol) glutaraldehyde in 0.15 M Sorensen buffer for 24 h. Briefly, cells were rinsed with cacodylate buffer, post fixed in osmium tetroxide, dehydrated in increasing concentrations of ethanol, and embedded in EPON resin (TAAB, Aldermaston, Bershire) while on coverslips. Ultrathin sections were contrasted with uranyl acetate and lead citrate and observed under an electron microscope (CM120; FEI). Micrographs were taken with a KeenView digital camera (Soft Imaging System).

**Both ampliseq transcriptome and Cancer hotspot panel v2.** The cDNA library was constructed using the Ion AmpliSeq Transcriptome Human Gene Expression kit or Ion Ampliseq Cancer Hotspot panel v2 and barcoded using and Ion Xpress Barcode Adapter (Thermofisher Scientific). The samples were quantified using Agilent High Sensitivity DNA kit and sequence on Ion Proton platform using Ion PI Hi-Q sequencing 200 kit chemistry. The sequencing results were aligned on hg19 and analysed with Partekflow (v6 Partek Inc) and Partek Genomic Suite or Torrent Suite Software.

**Soft Agar Assay for Colony Formation.** A Base 0.5% Agar was prepared under a top 0.3% Agar containing cells seeded at 25000/cm². Plates were incubated at 37°C in humidified incubator for 30 days. Cells were fed 1-2 times per week with cell culture media (DMEM+glutamax+10% FBS). At day 30, plates were stained with 0.5 ml of 0.005% Crystal Violet for 1 hour 30. Colonies were observed and counted under dissecting microscope.

**mFISH caryotype.** Caryotype analysis were performed on melanocytes by m-FISH analysis. Non-confluent cells were treated in melanocyte medium with colchicine at 20j.tg/mL (Eurobio) during 1h30 before hypotonic choc with 5.6mg/mL of KCl (Sigma Aldrich) and fixation. Analysis was made by m-FISH staining using mFISH 24Xcite probe (MetaSystems), according to the manufacturer's instructions. The metaphase's acquisitions were made with an Axiolmager Z2 microscope equipped with a camera cool cube and 10X and 63X plan apo objectives (Zeiss) piloted by Metafer software (Metasystems). The karyotype (30 to 50 metaphases per analysis) by m-FISH were then analyzed and classified with Isis software (Metasystems).

### RESULTS

### Characterization of a pure population of melanocytes derived from hPSC

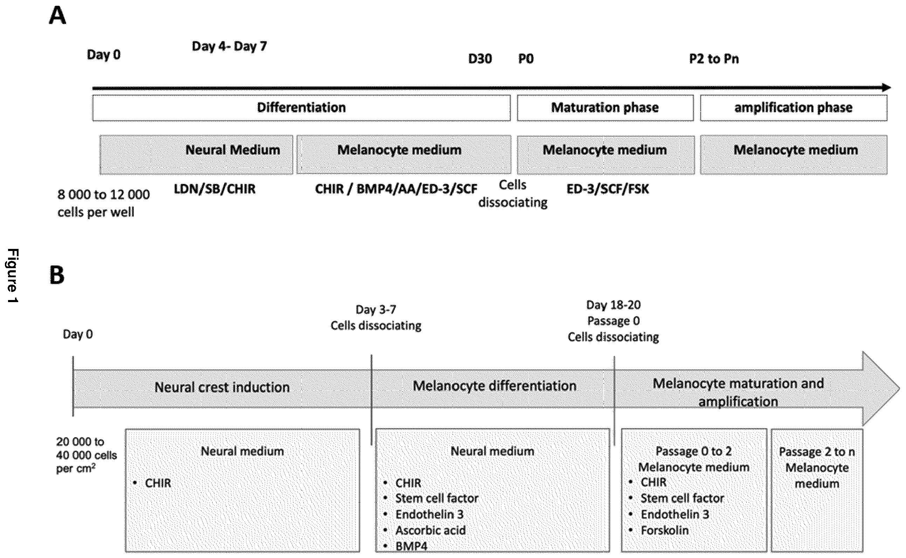

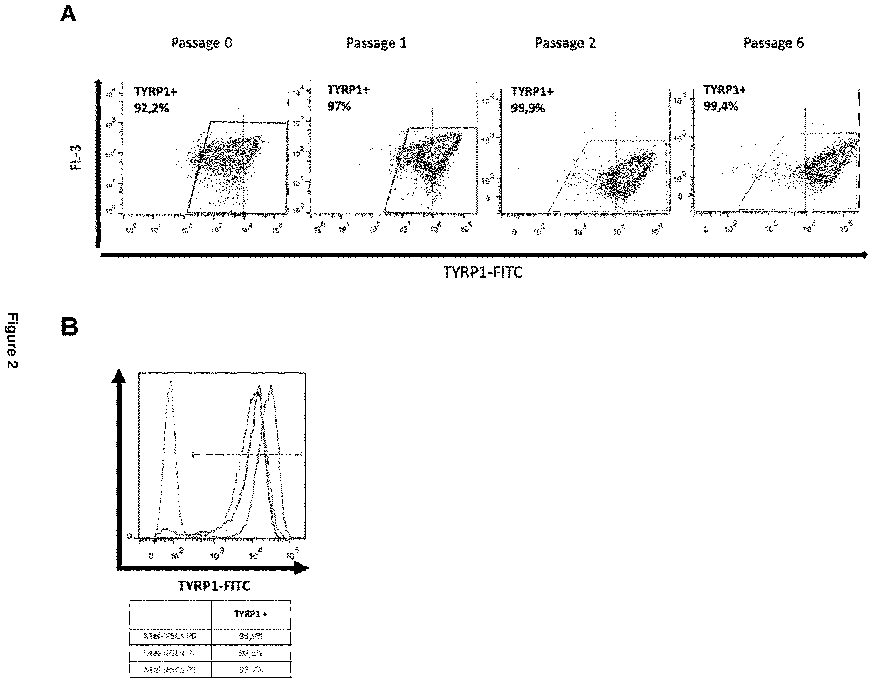

The present inventors have developed a multi-step protocol summarized in figure 1A. Basically, hPSC such as the hESC SA01 cell line have been aggregated in embryoid bodies (EBs). The induction of hPSC within the neural crest lineage was initiated by the use of dual-SMAD-inhibition in combination with Wnt signaling (Chambers et al. 2016; Gomez et al. 2019). At day 4, to initiate the melanocyte differentiation, small molecules including Endothelin-3 (EDN3), Stem Cell factor (SCF), Bone morphogenetic 4 (BMP4) and ascorbic acid (AA) required for the melanocyte precursor generation have been added in the medium at specific concentration (Nissan et al. 2011). At day 7, EBs were placed in a melanocytic medium (MGM-4, Lonza) with the same molecules to allow cells to exit and migrate out of the EBs. At day 30, to induce the maturation of melanocytes, the second phase, single dissociated cells were grown in MGM-4 supplemented with EDN3, SCF and Forskolin (FSK) essential to melanocyte survival and maturation. After two passages, the produced mature melanocytes are amplified (third phase) within MGM-4 medium. The cell morphology has been observed by microscopy analysis. EBs size increased during the first week of differentiation (day 5) after cell migration out of the EBs on day 10. From day 21 to day 30, cells acquire melanocyte-like dendritic cell morphology. After the dissociation step (Passage 0 (P0)), FSK is added in the medium to induce the maturation from P0 to passage 2 (P2). Homogenous melanocyte population (Mel-hPSC) are maintained in MGM-4 medium during the amplification phase showing a morphology comparable to the one observed in primary human melanocytes (HEMs). A molecular characterization was carried out and HEM was used as a positive control. Immunofluorescence staining of melanocytic markers including the main transcription factor of melanogenesis, MITF (microphthalmia associated transcription factor) and one of his targets, TYRP1 (tyrosinase-related protein 1) exhibited strong expression of both markers in Mel-hPSC. TYRP1 expression was confirmed by flow cytometry analysis showing positive cells greater than 99% (Figure 2A). A transcriptomic analysis was performed to compare the expression pattern of the Mel-hPSC genes with those of the HEM. The hierarchical clustering shows a similar genes expression profiling between Mel-hPSC, and HEM compared to hPSC. About 2000 genes have been found to be differentially upregulated in Mel-hPSCs and HEM compared to hPSCs (more than 70% are common). An enrichment analysis with GOBP (Gene ontology biological process) of Mel-hPSC shows a close genetic signature with the HEM. These common genes have a similar expression level.

### Functional melanocytes derived from hPSC

At the functional level, Mel-hPSC exhibit a clear pigmentation observed in cell pellets reflecting the synthesized melanin quantified by spectrophotometry. In addition, electron microscopy analysis showed the presence of all the four maturation stages of melanosome in the Mel-hPSC cytoplasm in the same manner as that observed in the HEM. Finally, an *in vitro* and *in vivo* 3D reconstructed skin was produced using Mel-hPSC. Basically, primary human keratinocytes (KHN) were added on the equivalent dermis with HEM or Mel-hPSC. They were expanded until confluency and were then maintained at the air-liquid interface in vitro for 14 days or grafted in vivo onto nude mice for two weeks. Macroscopical analysis revealed in vitro a well-pigmented epidermis under coculture conditions Mel-hPSC and KHN comparable to those observed under HEM and KHN. The distribution of melanocytes and keratinocytes in the epidermis was confirmed through TYRP1 and involucrin (IVL) staining, respectively. In parallel, the *in vivo* 3D reconstructed skin was analyzed showing two weeks after grafting a normal tissue architecture. As was demonstrated in the Fontana-Masson coloration, Mel-hPSC produce melanin as evidenced by TYRP1 expression stain and are in the basal layer of the epidermis, unstained by IVL.

### Genomic functional analysis during the differentiating and maturating process

In order to standardize our protocol, we sought to identify the molecular pathways that drive the genesis of human melanocytes derived from pluripotent stem cells using the next genome sequencing analysis. Expression pattern of the whole genome has been analyzed in a time dependent manner at day 0, day 7, day 14, day 21 and day 30 of the differentiation process using one hPSC cell line (hESC SA01 cell line). The principal component analysis (PCA) showed a similar transcriptional profile between replicates of each time point and highlighted the differences between time points. This finding was supported by the hierarchical clustering overview of the transcriptomic analysis. To decipher the molecular and cellular pathways involved in each stage of the developmental process, we performed bioinformatic analysis to group genes into clusters with a similar expression profile. Five clusters of genes were determined and analyzed by an enriched method using the ENRICH'R interface and in particular the enrichment tool, Gene Ontology (GO), describing the various biological processes, molecular and cellular mechanisms involved in each cluster. Cluster 1 included genes primarily related to embryonic development, morphogenesis and the development of epithelial tissues which are gradually repressed from day 0 to day 30. In the second cluster, genes linked to cell division and regulation of the cell cycle increased from day 0 to day 7 and show that expression decreases when cells migrate out of EB. Cluster 3 included several genes involved in the negative regulation of the embryonic development and neurogenesis, and the Wnt signaling pathway. Their expression has gradually increased from day 0 to reach a peak on day 14 before being repressed. Interestingly, the molecular signaling involved in melanogenesis is grouped into two clusters (4 and 5). These last two clusters regroup dozens of genes involved in melanosomal and cellular junction functions. 328 genes are related to the pigmentation pathway that showed a progressive increase between day 21 and day 30. To better decipher the cluster 5, a complementary analysis was carried out using STRINGdb interface which allow to perform a network with the known interactions between MITF, the master regulator of melanogenesis and other genes. 19 genes are directly connected to MITF, and functional annotation revealed 6 genes (circles colored in yellow) regrouped in the gene ontology term "developmental pigmentation. The size of the circles representing the degree of connectivity with MITF. This analysis highlights SOX10, DCT, TYR and RAB27. To confirm our data, the gene expression profiles identified were validated by quantitative real-time PCR.

After the dissociation step (Passage 0 (P0)), FSK is added in the medium to induce the maturation from P0 to passage 2 (P2). Homogenous melanocyte population (Mel-hPSC) are maintained in MGM-4 medium during the amplification phase showing a morphology comparable to the one observed in primary human melanocytes (HEMs)

Next, we sought to better characterize the maturation phase induced by FSK treatment. Additional genomic sequencing analysis was carried out to compare the expression profile of the Mel-hPSC genes at passage 4 (P4) (mature Mel-hPSC) compared to passage 0 (P0) (immature Mel-hPSCs). Our analysis showed a differential expression gene pattern at P4 in comparison with P0. Many differentially expressed genes (DEGs) were identified (pValue adjusted ≤ 0.5; fold change ≥ 1.5; minReads100). There were 1309 upregulated and 1719 downregulated DEGs. When upregulated DEGs were mapped to an enrichment tool, the top three significantly enriched gene ontology categories were "lytic vacuole," "lysosome" and "melanosome". qPCR analysis for *MITF* and its target genes (*TYPR1*, *TYR* and *PMEL17*) was assessed in Mel-hPSC of P0 and P1 referred as immature stage and compared with Mel-hPSC of P2, P3 and P6 referred to mature stage showing a gradual increase of melanogenesis genes. In addition, western blot analysis has shown the major enzyme involved in melanin synthesis, tyrosinase is not express at passage 0 and gradually becomes express after passages. Similarly, flow cytometry analysis has shown that over 90% of immature Mel-hPSC are TYRP1-positive with a difference in expression intensity compared to mature Mel-hPSC. This data is confirmed by the melanin synthesis observed from passage 2 to 6.

### Long-term behavior of mel-hPSC.

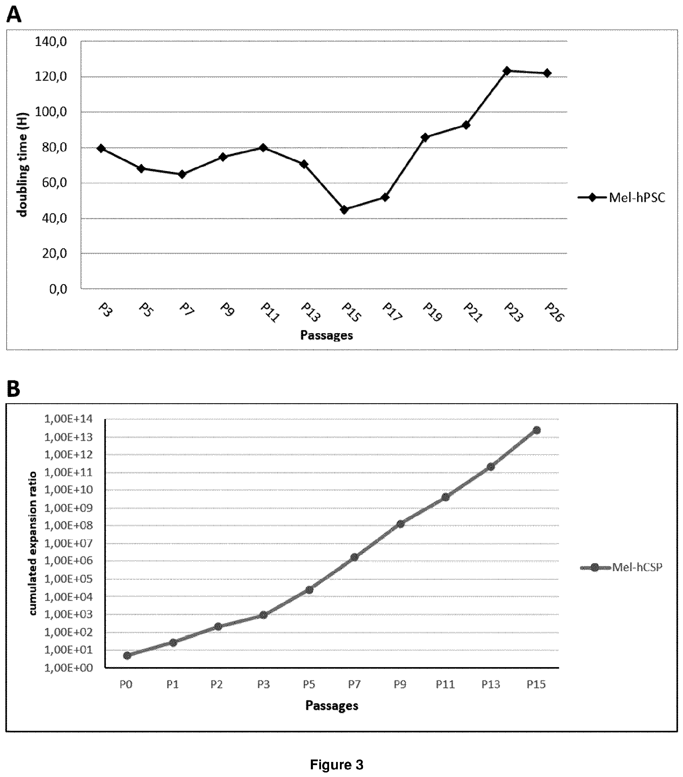

For large drug screening campaign and cellular therapy approaches, a large number of melanocytes is needed. Melanocyte proliferation capacity have been tested by measuring the doubling time at early (from passage 2 to passage 15), and late passages (>passage 15) in mel-hPSC 5 (Figure 3). At the early passages, both, Mel-hPSC maintained a stable doubling time at 60 hours. The late passages showed a slight increase in their doubling about 100 hours. At passage 26, doubling time reaches to 120 hours for Mel-hESC. Furthermore, we have showed that over-time in culture, Mel-hPSC expressed all melanocytic markers as shown in the qPCR analysis, immunostaining, FACS and immunoblots analysis and maintained dendritic morphology. To verify that Mel-hPSC have not been transformed into malignant cells over-time in culture, an in vitro soft agar assay was assessed on Mel-hPSC at early and late passages. We used one melanoma cell line (SKMEL28) as a positive control. As predicted, the formation of large colonies is observed in the melanoma cell line, while no colonies have been detected in Mel-hPSC and HEM. Karyotype analysis of late passages revealed no chromosomal abnormalities. Mutations of 50 oncogenic and tumor suppressor genes were analyzed using the Ampliseq cancer hotspots library in Mel-hPSC at early and late passages. The melanoma cell line (SKMEL28) shows the pathogenic mutations in EGFR, BRAF and PTEN, already described for this cell line. For the Mel-hPSC, the SNPs detected are referred as neutral mutations by the FATHMM database. All identified SNPs were positioned either in intronic regions or induce silent coding.

### Conclusions

In this study, we have established a standardized protocol in several steps to generate from hPSC, a pure melanocyte population. A temporal genome-wide transcriptome analysis was used to conduct and validate the differentiation process. The functional melanocytes generated have all the characteristic of primary melanocytes and are maintained in long-term in culture without losing their phenotype.

Combining the principal component analysis, clustering, and correlation analyses of the RNA-seq data, several clusters of genes have been identified that recapitulate the loss of pluripotency, the neural crest induction, and the melanocyte specification. The resulting melanocytes exhibited homology to adult melanocytes. Interestingly, the highly upregulated process has been related to the canonical Wnt signaling pathway important for neural crest induction and for the melanocytic lineage induction at the expense of neuronal lineage (Hari et al. 2012; Dunn et al. 2005). The process of differentiation of melanocytes was confirmed by a signature of genes all related to melanogenesis and to the biogenesis and transport of melanosomes. Computational analysis has shown that most genes are associated with MITF, the master regulator of melanogenesis. Overall, this system provides important basic information on the proper conduct of the differentiation and can be used as a quality control. In addition, this data set would make it possible to identify specific human molecular signatures that represent the chronobiological development of human melanocytes which have so far been mainly studied in mouse models (Cichorek et al. 2013).

Meanwhile, a maturation step was identified at the end of the differentiation. To demonstrate the cellular process involved in this maturation process, the gene expression profiling of mature melanocytes (passage 4) was compared with immature melanocytes (Passage 0). Transcriptomic analysis revealed a group of genes up regulated in mature melanocytes and mainly involved in melanosomes, lysosomes, and pigment granules biological functions. These data are consistent with the already well-described cellular process in the pigmentation process. Melanosome maturation, movement and transport are regulated by several genes including the Small GTPases Rab family (Homma, Hiragi, and Fukuda 2021). To illustrate our findings, we identified *Rab27a* and *Myosin-Va* to be upregulated in the mature melanocytes compared to the immature ones. Both have a major role in the peripheral distribution of melanosomes (Hume et al. 2001). Our analysis highlighted several other Rab isoforms including *Rab42*/*7B*, *Rab4a* et *Rab7a* to be also upregulated in mature melanocytes known to be involved in the regulation of melanosome biogenesis (Marubashi and Fukuda 2020). In addition, we showed an upregulation of the main melanogenesis enzymes, *Tyrosinase* and *TYRP1* that are required for melanin synthesis. Their intracellular transport required *Rab7* also identified in our analysis (Hirosaki et al. 2002). Several other Rab family isoforms have been found to be downregulated (*Rab6b*, *Rab39b, Rab3a, Rab33a, Rab31*) or upregulated (*Rab3d*, *Rab40b*) in mature melanocytes. Their function in melanosome biology remains unclear. As the dysfunction of Rab isoforms causes human hereditary disease the identification of new fundamental regulators of melanosome players would ultimately lead to a better understanding of the skin pigmentation.

The most common acquired depigmentation disorder is vitiligo affecting more than 70 million people worldwide. Whereas this disease is often dismissed as a cosmetic problem, vitiligo can be psychologically devastating, often with a considerable burden on daily life. Vitiligo is clinically characterized by depigmented macules resulting from the loss of functional melanocytes. To date, current therapies are limited, and no known treatment can consistently induce repigmentation in all patients. Furthermore, there are patients who are not responsive to medical procedures (Khaitan, Kathuria, and Ramam 2012) and for whom surgery is the only option. Surgical therapy consists in the introduction of missing melanocytes in the depigmented lesions from pigmented skin of the same person by different procedures (e.g., tissue grafts or cellular grafts). However, this technique can induce depletion of site donor. So far, the maintenance of mel-hPSC has been difficult. Cell proliferation capacity diminish with passages of cells. After eight to ten generations in culture, the cells begin to proliferate slowly and become differentiated (Tang et al. 2014). By improving the culture conditions, we can maintain the mel-hPSC in long-term in culture without any senescence observed (>20 passages). That's a critical point for clinical perspectives who request a large scale-up production of melanocytes. Accordingly, the use of human hPSC-derived melanocytes may prove invaluable in developing a novel cell therapy for patients with vitiligo. The present standardized and well characterized system to generate melanocytes from hPSCs will be useful to improve the knowledge of mechanisms involved in the development of pigmentation disorders and further for developing grade clinic melanocytes for cell-based therapeutic applications.

### Example 2: 2D Method for the large-scale production of melanocytes derived from human pluripotent stem cells

Differentiation into melanocytes was initiated according to the protocol summarized in Figure 1B. The first step was to induce the neural crest (NC) lineage, the embryonic origin of melanocytes, by the addition of CHIR99021, which functions as an activator of the (WNT) pathway, participating in the induction of neural crest cells (NCCs) with the Bone Morphogenic Protein (BMP) pathway. Melanocyte differentiation is then induced by the addition of growth factors including endothelin 3 (EDN3), Stem Cell Factor (SCF), BMP4 (Bone Morphogenic Protein 4), and ascorbic acid (AA), at well-defined concentrations. The 254CF medium is a medium for the survival and maturation of melanocyte.

More specifically, hPSC cells (such as hiPSC 1503 cell line) are seeded on a Matrigel^{®} support in DMEM/F12+Glutamax medium supplemented with B27 woVitA 2% and BSA 0.5% (Bovine Serum Albumin 25%) with the addition of Rock Inhibitor at 10 µM and CHIR99021 at 3µM. The medium is changed every day for 5 days with the extemporaneous addition of CHIR99021 at 3µM. At day 7, cells are dissociated using Accutase^{®}. The cells are then reseeded on matrigel support in the same medium with the addition of 3µM CHIR99021, 50ng/mL SCF, 100nM END3, 0.3mM ascorbic acid and 10.9pM BMP4. The medium is changed every 2-3 days with the same cytokines, until their dissociation after 18-20 days. The cells are then reseeded at 30000 cell/cm² in 254CF medium (Human Melanocyte Basal Medium), supplemented with the same cytokines and Forskolin without coating. The cells are then amplified in 254CF medium alone.
Melanocytes, derived from differentiation of hPSC are cultured in 254CF medium. Cells are passaged in trypsin (0.05%Trypsin-EDTA, Invitrogen) diluted to ½ in PBS and reseeded at 10000 cells /cm² in 254CF medium without coating.

### Method

Immunofluorescence (IF). Cells (keratinocytes and melanocytes) are seeded in slide-mounted culture chambers (Lab-Tek^{®}II Chamber Slide^{™}) on a collagen support. Cells are fixed in 4% Paraformaldehyde (PFA, Euromedex) and then permeabilized in 0.1% Triton (Euromedex) solution. A saturation of the aspecific sites is then performed with a solution of PBS/BSA 1% (Bovine Serum Albumin; Sigma). Primary antibodies (see Appendix 1) are diluted in a solution of PBS + 2% goat serum (Invitrogen) and incubated for 1 hour at room temperature or overnight at 4°C. The secondary antibodies, diluted in 2% PBS+ goat serum solution and mixed with a nuclear marker DAPI (1/5000, H3569 Invitrogen) are then incubated 30 minutes at room temperature. Slides are mounted with FluoroMount (FluoroMount Southern Biotech) and photos are taken using a Zeiss microscope equipped with epifluorescence illumination.

Flow cytometry. Melanocytes and iPSCs are dissociated with trypsin (0.05%Trypsin-EDTA, Invitrogen) diluted at 1/2 and Accutase^{®} (StemPro^{®} Accutase^{®}, Gibco) respectively. The cells are then fixed with cytofix (BD Cytofix/Cytoperm, BD Biosciences) according to the manufacturer's recommendations. Fluorochrome-conjugated antibodies are diluted in a solution of PBS-SVF 2% + 10% cytoperm (BD Perm/Wash, BD Biosciences) and incubated for 30 minutes in the dark at room temperature. Analyses are performed with the MACSQuant system (MiltenyiBiotec) and the Flowjo software.

Melanin assay. Cell pellets of 200 000 cells are dissociated in 1N NaOH. The absorbance reading at 405nm is performed on the Clariostar microplate reader (BMG Labtech). In parallel, a standard range of synthetic melanin is performed to allow the calculation of melanin concentrations in pg/mL.

### Results

In order to characterize the mel-hPSC, phenotypic analyses were performed to verify the protein expression of melanocyte markers. TYRP1, one of the key enzymes of melanogenesis was analyzed by flow cytometry. The results show a TYRP1 positive population of more than 99% for differentiated mel-hPSC (Figure 2B).

Immunofluorescence analyses of the melanocyte markers including MITF, PMEL17, DCT and TYR confirmed the results obtained by flow cytometry. Co-staining of PME17L and MITF was performed. Cytoplasmic expression of PMEL17 and nuclear expression of MITF are observed. In addition, the mel-hPSC expressed the main enzyme of melanogenesis, tyrosinase and DCT.

To characterize the mel-hPSC at the functional level, melanin measurements were performed. Macroscopic observation of the cell pellet at each passage indicates by its color whether melanin is produced in the mel-hPSC. The observed pigmentation correlates with the results obtained for the melanin assay.

As observed in the 3D protocol, a maturation phase of melanocytes between P0 and P2 has been also observed in the 2D protocol. Melanogenesis is analyzed through the expression of one of its markers, TYRP1, by flow cytometry. It is observed that at the end of differentiation (D18), cells do not express TYRP1. At P0, we observe a population positive for TYRP1 at 93.9% with an increase in the intensity of expression with each passage (P0 to P2).

The pigmentation of the cells between P0 and P2 was assessed by observation of the cell pellets and by melanin assay. Cell pellets of mel-hPSC were much more pigmented at P2 than at P0 or P1. This observation was confirmed by the melanin assay at different passages. The addition of forskolin in the media, induced the maturation of melanocytes.

### Conclusions

In this study, we showed an easy-to-use method to differentiate human pluripotent stem cell (hPSC) into melanocytes. To obtain a pure population of melanocytes, most of the previous protocols required mechanical or fluorescent-activated cell sorting with a specific cell surface marker to specifically isolate the melanocyte population. In our method, we generated a pure and homogenous population of melanocytes without any selection. The mature mel-hPSC generated showed a comparable morphology to primary human melanocytes and expressed all the melanogenesis markers. To test their functionality, *in vitro* experiments was conducted. More specifically, the generated mel-hPSCs produced melanin and can transfer melanosome to keratinocytes. This easy-to-use and standardized protocol will facilitate its adaptation into a clinical grade hPSC protocol for cellular therapy applications.

## Claims

1. An *in vitro* method for obtaining a population of human melanocytes, wherein said method comprises a differentiation step wherein neural crest cells are cultured in presence of a Glycogen Synthase Kinase 3 (GSK3) inhibitor such as CHIR99021 or Wingless-type family member 3A WNT3a, Bone Morphogenic Protein 4 (BMP4), Ascorbic Acid (AA), Stem Cell Factor (SCF) and Endothelin 3 (EDN3).

2. The method according to claim 1, wherein said method comprises the following steps:
a) an induction step wherein human pluripotent stem cells are cultured in a neural medium to obtain neural crest cells;
b) a differentiation step wherein the neural crest cells obtained in step a) are cultured in presence of a GSK3 inhibitor such as CHIR99021 or WNT3a, BMP4, AA, SCF and EDN3 to obtain melanocyte precursors;
c) a maturation step wherein the melanocyte precursors obtained in step b) are cultured in presence of at least one agent stimulating the maturation of melanocytes to obtain mature human melanocytes; and
d) an amplification step wherein the melanocytes obtained in step c) are cultured on a medium suitable for melanocyte culture to obtain a population of human melanocytes.

3. The method according to claim 2, wherein step a) is carried over 3 to 7 days.

4. The method according to claim 2 or 3, wherein said method further comprises a step b'), wherein the melanocyte precursors obtained in step b) are enzymatically sorted prior to step c).

5. The method according to any one of claims claim 2 to 4, wherein said agent stimulating the maturation of melanocytes is an activator of the cAMP pathway such as forskolin.

6. The method according to any one of claims 2 to 5, wherein said method is carried out on a 2D cell culture environment.

7. The method according to claim 6, wherein said human pluripotent stem cells are seeded at a concentration of 20 000 to 40 000 cells per cm².

8. The method according to claim 6 or 7, wherein said neural medium comprises CHIR99021 or WNT3A.

9. The method according to any one of claims 6 to 8, wherein said method further comprises a step a'), wherein the neural crest cells obtained in step a) are enzymatically sorted prior to step b)

10. The method according to any one of claims 6 to 9, wherein step b) is carried over 10 to 15 days.

11. The method according to any one of claims 6 to 10, wherein said method comprises the following steps:
a) culturing human pluripotent stem cells at a concentration of 20 000 to 40 000 cells per cm² on a neural culture medium comprising CHIR99021 during 3 to 7 days to obtain neural crest cells;
a') enzymatically sorting the neural crest cells obtained in step a);
b) culturing the neural crest cells sorted in step a') on the neural culture medium further comprising BMP4, AA, SCF and EDN3 during 10 to 15 days to obtain melanocyte precursors;
b') enzymatically sorting the melanocyte precursors obtained in step b);
c) culturing the melanocyte precursors sorted in step b') on the culture medium used in step b) further comprising forskolin and passaging the cells once or twice to obtain mature human melanocytes; and
d) culturing the melanocytes obtained in step c) on a medium suitable for melanocyte culture during at least 20 passages to allow for their proliferation and obtain a population of human melanocytes.

12. The method according to any one of claims 2 to 5, wherein said method is carried out in a 3D cell culture environment.

13. The method according to claim 12, wherein said 3D cell culture environment is a 96-well plate, and wherein said human pluripotent stem cells are seeded at a concentration of 8 000 to 12 000 cells per well.

14. The method according to claim 12 or 13, wherein said neural medium comprises CHIR99021 or WNT3a, LDN193189 and SB431542.

15. The method according to any one of claims 12 to 14, wherein step b) is carried over 20 to 28 days.

16. The method according to any one of claims 6 to 10, wherein said method comprises the following steps:
a) culturing human pluripotent stem cells on a 96-well plate at a concentration of 8 000 to 12 cells per well in a neural culture medium comprising CHIR99021, LDN193189 and SB431542 during 3 to 7 days to form embryoid bodies (EB) containing neural crest cells;
b) plating the embryoid bodies obtained in step in step a) to allow the neural crest cells to exit said embryoid bodies and then culturing during 20 to 25 days said neural crest cells in a medium suitable for melanocyte culture further comprising CHIR99021, BMP4, AA, SCF and EDN3 to obtain melanocyte precursors;
b') enzymatically sorting the melanocyte precursors obtained in step b);
c) culturing the melanocyte precursors sorted in step b') on the culture medium used in step b) further comprising forskolin to obtain mature human melanocytes; and
d) culturing the melanocytes obtained in step c) on a medium suitable for melanocyte culture during at least 20 passages to allow for their proliferation and obtain a population of human melanocytes.

17. A population of human melanocytes, wherein said population is obtainable by means of the method according to any one of claims 1 to 16.

18. The population of human melanocytes according to claim 17 for use in the treatment of a skin pigmentation disorder, such as vitiligo, or in the treatment of a skin lesion.

19. Use of the population of human melanocytes according to claim 17 as a cellular model.

20. A method for screening candidate cosmetic or therapeutic compounds, wherein said method comprises the following steps:
i) Culturing the population of human melanocytes according to claim 17 in presence of a candidate compound;
ii) Assaying the effects of said candidate therapeutic compound on said population of human melanocytes; and
iii) Selecting said compound based on said effects on the population of human melanocytes.
